Europäisches Patentamt

European Patent Office

Office européen des brevets

(19)

(11) Veröffentlichungsnummer: **0 118 008**
**A2**

# (12) EUROPÄISCHE PATENTANMELDUNG

(21) Anmeldenummer: **84100833.7**

(22) Anmeldetag: **26.01.84**

(51) Int. Cl.³: **A 61 M 5/14**

(30) Priorität: **31.01.83 DE 3303102**

(43) Veröffentlichungstag der Anmeldung:
**12.09.84 Patentblatt 84/37**

(84) Benannte Vertragsstaaten:
**DE FR GB SE**

(71) Anmelder: **Siemens-Elema AB**
**Röntgenvägen 2**
**S-171 95 Solna 1(SE)**

(84) Benannte Vertragsstaaten:
**SE**

(71) Anmelder: **SIEMENS AKTIENGESELLSCHAFT**
**Berlin und München Wittelsbacherplatz 2**
**D-8000 München 2(DE)**

(84) Benannte Vertragsstaaten:
**DE FR GB**

(72) Erfinder: **Cewers, Göran**
**Moellevongsvaegen 89**
**S-222 40 Lund(SE)**

(72) Erfinder: **Olsson, Sven-Gunnar, Dipl.-Ing.**
**Postläda 652**
**S-240 17 Soedra Sandby(SE)**

(54) **Anordnung zur Regelung der Durchflussmenge in einem Infusionsgerät.**

(57) Um bei Anordnungen zur Regelung der Durchflussmenge und insbesondere bei Infusionsgeräten die Sicherheit weiter zu erhöhen, sind erfindungsgemäss zwei unabhängig voneinander arbeitende Mikroprozessoren (1,2) vorgesehen, denen Soll- und Ist-Werte parallel zugeführt werden und die bestimmte Parameter untereinander austauschen und vergleichen. Bei Abweichungen wird als Schutzmassnahme beispielsweise ein Nonstopp (7) ausgelöst. Weitere Funktionen der Anordnung können doppelt ausgeführt sein oder Funktionskontrollen aufweisen.

SIEMENS AKTIENGESELLSCHAFT

Berlin und München

Unser Zeichen

VPA 83 P 8506 E

0118008

BEZEICHNUNG GEÄNDERT
siehe Titelseite

Anordnung zur Regelung der Durchflussmenge

Die Erfindung betrifft eine Anordnung zur automatischen Regelung der Durchflussmenge in einem Infusionsgerät mit Mitteln zum Einstellen vorgebbarer Sollwerte für den Flüssigkeitsdurchsatz pro Zeiteinheit, die totale zuzuführende Flüssigkeitsmenge, die Art der Flüssigkeit und gegebenenfalls die Art der verwendeten Tropfenkammer, mit einem Tropfendetektor als Istwertgeber, einem Einstellorgan für die Tropfengeschwindigkeit sowie einer Steuereinheit, die aus Soll- und Istwerten eine Regelgrösse für das Einstellorgan ermittelt. Eine derartige Anordnung ist beispielsweise aus der US-PS 4 294 248 bekannt.

Der vorliegenden Erfindung liegt die Aufgabe zugrunde, die Funktion einer derartigen Anordnung ohne grossen Aufwand wesentlich sicherer zu machen, um Risiken bei der Behandlung von Patienten weitgehend auszuschliessen.

Diese Aufgabe wird erfindungsgemäss dadurch gelöst, dass zwei unabhängig voneinander arbeitende Mikro-Prozessoren vorgesehen sind, denen beiden sowohl die Ist- als auch die Sollwerte zugeführt sind, dass mindestens ein Mikroprozessor als Steuereinheit dient, dass beide Mikro-Prozessoren über eine Datenleitung miteinander verbunden sind zum Austauschen bestimmter Parameter, dass in jedem Mikro-Prozessor ein Vergleich des jeweils empfangenen Parameters mit dem eigenen durchgeführt wird und dass ab einer bestimmten, vor-

Gdl 1 Een / 31.1.1983

gebbaren Abweichung eine Schutzmassnahme ausgelöst wird.

Allein bereits durch die Verwendung zweier Mikro-Prozessoren, die sich praktisch gegenseitig kontrollieren, wird sichergestellt, dass eine grosse Anzahl möglicherweise auftretender Fehler nicht zu einer falschen Durchflussmenge führen, ohne das zumindesten ein Alarmsignal abgegeben wird. So führen beispielsweise Fehler in der Datenübertragung von den Einstellmitteln für die Ist- und Sollwerte oder Fehler in der Signalübertragung vom Tropfendetektor, Fehler in der Erkennung der empfangenen Signale oder auch Fehler in deren Verarbeitung bereits nach kurzer Zeit zum Auslösen einer Schutzmassnahme.

Zur weiteren Erhöhung der Sicherheit der gesamten Anordnung ist vorgesehen, dass die Mittel zum Einstellen der Sollwerte dubliert sind, insbesondere dass diese einmal einfach kodiert und einmal komplementär kodiert erzeugt werden. Nur wenn der zweite Wert tatsächlich das Komplement des ersten ist, wird der übertragende Sollwert von den Mikroprozessoren als richtig anerkannt und übernommen.

Eine wesentliche Erhöhung der Sicherheit ergibt sich noch dadurch, dass als Schutzmassnahme ein Notstopp zur vollständigen Unterbindung des Flüssigkeitsdurchsatzes vorgesehen ist. Auch hier ist es wieder besonders vorteilhaft, dass dieser Notstopp durch beide Mikroprozessoren auslösbar ist. Selbst, wenn einer dieser Mikroprozessoren teilweise oder ganz ausfallen sollte, kann der Flüssigkeitsdurchsatz immer noch unterbrochen werden.Weiterhin kann das Einstellorgan für die Tropfengeschwindigkeit praktisch als Duplikat für den Notstopp ausgelegt sein.

Um sicher zu verhindern, dass sich möglicherweise in der Flüssigkeit befindende Gasblasen zum Patienten gelangen, ist ein Blasendetektor vorgesehen, dessen Signal ebenfalls auf beide Mikroprozessoren gegeben ist und der beim Detektieren einer Gasblase das Auslösen des Notstoppes verursacht.

Um insbesondere auch bei transportablen Anordnungen stets ein einwandfreies Funktionieren sicherstellen zu können, sind als Energieversorgung zwei unabhängige Batterien vorgesehen. Ueber entsprechende Indikatoren können die Ladezustände dieser Batterien wiederum auf die Mikroprozessoren gegeben werden. Als besonders vorteilhaft hat es sich dabei erwiesen, dass beim Ausfall einer Batterie ein Warnsignal erzeugt wird, und erst beim Ausfallen beider Batterien Alarm gegeben wird und/oder der Notstopp ausgelöst wird. Dazu kann der Notstopp vorteilhafterweise mit einer Hilfsspannungsquelle versehen sein, durch die er auch beim Ausfall der Batterien betätigbar ist.

Weitere Vorteile der Erfindung ergeben sich aus den übrigen Unteransprüchen.

Anhand eines Blockschaltbildes wird im folgenden ein Ausführungsbeispiel der Erfindung näher beschrieben und erläutert.

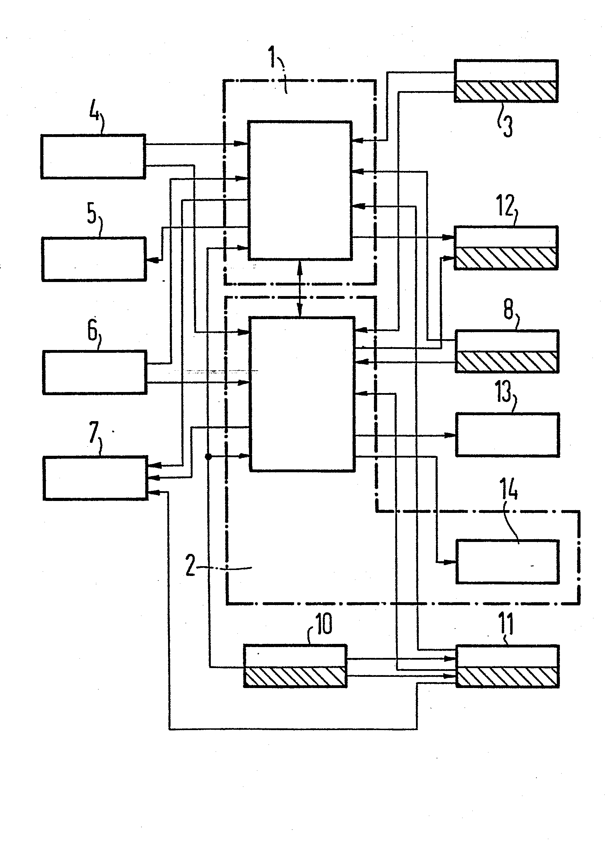

In der einzigen Figur sind schematisch die wesentlichsten, die Erfindung betreffenden Teile eines Infusionsgerätes dargestellt. Hauptbestandteil der Anordnung zur automatischen Regelung der Durchflussmenge sind zwei Mikroprozessoren 1 bzw. 2, die die Kontroll,-Steu- erungs- und Ueberwachungsfunktionen ausführen. Weiterhin sind Einstellmittel 3 zur Eingabe von Sollwerten in die Mikroprozessoren dargestellt. Bei diesen Einstellmitteln kann es sich beispielsweise um mechanisch betätigbare Schalter wie Daumenräder handeln, deren Werte digital kodiert auf die Mikroprozessoren gegeben werden. Verwendet man zum Kondieren beispielsweise einen BCD-Code, so sind dazu vier Bit notwendig. Da die Datenbusleitungen zu den Mikroprozessoren üblicherweise acht Bit übertragen könnnen, ist es auf einfache Weise möglich, neben dem direkt kodierten Wert auch noch dessen Komplement zu übertragen und damit gleichzeitig eine Sicherheitskontrolle zu haben. Nur wenn die beiden vierstelligen Binärzahlen komplementär zueinander sind, wird der übertragene Wert von den Mikroprozessoren als korrekt angenommen. Auf diese Weise kann unterstellt werden, dass die Einstellmittel quasi doppelt vorhanden sind. Das ist dadurch angedeutet, dass der entsprechende Block in der unteren Hälfte schraffiert ist. Weiterhin ist ein Tropfendetektor 4 vorgesehen, dessen Signale ebenfalls auf beide Mikroprozessoren 1 und 2 gegeben sind. Im vorliegenden Ausführungsbeispiel ist der Tropfendetektor nur einfach vorgesehen. Der Tropfendetektor kann beispielsweise nach optischen Prinzipien arbeiten und dazu mit einer Leuchtdiode und einer Photodiode ausgestattet sein. Die Funktionskontrolle kann in einem derartigen Fall zumindestens das einwandfreie Funbktionieren dieser Elemente beinhalten, d.h, der Ausfall eines dieser Elemente sollte dann zumindest als Fehler angezeigt werden.

Weiterhin ist ein Einstellorgan 5 vorgesehen, das beispielsweise aus einem motorgetriebenen Exzenter bestehen kann, an dem ein Schlauch für die Flüssigkeit vorbeigeführt ist. Dieses Einstellorgan wird über den Mikroprozessor 1, der die Steuerfunktionen ausführt, geregelt.

Weiterhin ist ein Blasendetektor 6 vorgesehen, dessen Signale auf beide Mikroprozessoren gegeben werden und der wiederum ebenso wie der Tropfendetektor mit einer Funktionskontrolle versehen sein kann. Als Notstopp 7 kann vorteilhafterweise ein federbelasteter Hebel verwendet werden oder auch ein entsprechender Exzenter, der energiesparend mit geringer Federkraft in Sperrstellung gehalten wird und beim Auslösen mit grosser Federkraft den Schlauch für die Flüssigkeit abklemmt. Der Notstopp ist von beiden Mikroprozessoren betätigbar, so dass sichergestellt ist, dass auch beim teilweisen oder vollständigen Ausfall eines dieser Mikroprozessoren noch ein Unterbinden des Flüssigkeitstransportes möglich ist.

Im vorliegenden Ausführungsbeispiel ist der Notstopp zwar nur einfach ausgeführt, jedoch kann zumindestens teilweise das Einstellorgan 5 als Ersatz für den Notstopp angesehen werden. Selbstverständlich ist es auch denkbar, dass der Notstopp direkt doppelt ausgeführt ist.

Neben den Einstellmitteln 3 ist im vorliegenden Ausführungsbeispiel auch noch eine Fernsteuerung 8 vorgesehen, die ebenso wie die Einstellmittel 3 durch spezielle Kodierung mit einer doppelten Sicherheit versehen ist, was wiederum durch die Schraffierung im unteren Teil des Blockes 8 angedeutet ist. Die über die Fernsteuerung 8 einzugebenden Werte gelangen ebenfalls auf beide Mikroprozessoren.

Doppelt ausgeführt ist weiterhin die Batterie 10, die als Energieversorgung für die gesamte Anordnung dient. Der Uebersichtlichkeit halber ist hierbei lediglich die Energieversorgung der Mikroprozessoren 1 und 2 durch Pfeile angedeutet, nicht jedoch die der anderen Elemente, die wie die Mikroprozessoren von beiden Batterien versorgt werden.

Der Ladezustand der Batterien 10 wird über einen Batterieindikator 11 überwacht, der wiederum an beide Mikroprozessoren angeschlossen ist. Fällt beispielsweise nur eine Batterie aus oder sinkt die Ladespannung einer Batterie unter einen vorgegebenen Grenzwert, so erzeugt die Anordnung ein hier nicht näher dargestelltes Warnsignal beispielsweise in Form einer aufleuchtenden Lampe. Fallen hingegen beide Batterien aus, so wird über die Mikroprozessoren ein Alarm 12 ausgelöst, der sowohl optisch als auch akustisch sein kann. Gleichzeitig wird der Notstopp 7 ausgelöst.

Schliesslich enthält die Anordnung ein Display 13 und einen Speicher 14, der Teil des Mikroprozessors 2 sein kann und in dem mindestens die totale Durchflussmenge zu veschiedenen Zeitpunkten gespeichert werden kann.

Mit der vorliegenden Anordnung werden praktisch sämtliche auftretenden Störfälle derart berücksichtigt, dass eine Gefahr für den Patienten durch eine unkontrolliert grosse Menge von Infusionsflüssigkeit nicht entstehen kann.

Ohne den Rahmen der vorliegenden Erfindung zu verlassen, sind selbstverständlich eine grosse Reihe von Variationen möglich. So kann beispielsweise auf die Fernsteuerung oder die Einstellung der Mittel verzichtet werden und ebenso auf den Speicher oder das Display. Auch kann es unter Umständen genügen, eine Batterie zu verwenden oder bei stationären Geräten einen Netzanschluss zu verwenden. Weiterhin wäre es beispielsweise auch denkbar, nur das Einstellorgan gleichzeitig als Notstopp einzusetzen. Bei all diesen Variationen sollte aber sichergestellt sein, dass stets nach kurzer Zeit nach dem Auftreten eines Fehlers zumindest Alarm gegeben wird oder zumindest eine Schutzmassnahme wie der Notstopp ausgelöst wird. Vorteilhafterweise kann absichtlich die Anordnung eine gewisse Trägheit gegenüber auftretenden Fehlern aufweisen, um beispielsweise beim Verstellen mechanische Einstellmittel beim Uebergang vom einen Einstellwert zum anderen nicht bereits den kurzzeitig undefinierten Wert als Fehler auszuweisen und evtl. Alarm auszulösen. Weiterhin können auch diejenigen Parameter, die beim Austausch der Werte zwischen den beiden Mikroprozessoren untereinander verglichen werden, variieren.

15 Ansprüche
1 Figur .

C118008

## Patentansprüche

1. Anordnung zur automatischen Regelung der Durchflussmenge in einem Infusionsgerät mit Mitteln zum Einstellen vorgebbarer Sollwerte für den Flüssigkeitsdurchsatz pro Zeiteinheit, die totale zuzuführende Flüssigkeitsmenge, die Art der Flüssigkeit und gegebenenfalls die Art der verwendeten Tropfenkammer, mit einem Tropfendetektor als Istwertgeger, ein Einstellorgan für die Tropfengeschwindigkeit sowie einer Steuereinheit, die aus Soll- und Istwerten eine Regelgrösse für das Einstellorgan ermittelt, d a d u r c h g e k e n n - z e i c h n e t, dass zwei unabhängig voneinander arbeitende Mikroprozessoren (1,2) vorgesehen sind, denen beiden sowohl die Ist- als auch die Sollwerte zugeführt sind, dass mindestens ein Mikroprozessor (1) als Steuereinheit dient, dass beide Mikroprozessoren (1,2) über eine Datenleitung miteinander verbunden sind zum Austauschen bestimmter Parameter, dass in jedem Mikroprozessor (1,2) ein Vergleich des jeweils empfangenen Parameters mit dem eigenen durchgeführt wird und dass ab einer bestimmten,vorgebbaren Abweichung eine Schutzmassnahme ausgelöst wird.

2. Anordnung nach Anspruch 1, d a d u r c h g e - k e n n z e i c h n e t, dass weitere Funktionen doppelt ausgeführt sind.

3. Anordnung nach Anspruch 2, d a d u r c h g e - k e n n z e i c h n e t, dass die Mittel (3) zum Einstellen der Sollwerte dubliert sind.

4. Anordnung nach Anspruch 3, d a d u r c h g e - k e n n z e i c h n e t , dass die Sollwerte einmal einfach kodiert und einmal koplementär kodiert erzeugt werden.

C118008

5. Anordnung nach einem der Ansprüche 1 bis 4, d a d u r c h  g e k e n n z e i c h n e t, dass als eine Schutzmassnahme ein Notstopp (7) zur vollständigen Unterbindung des Flüssigkeitsdurchsatzes vorgesehen ist.

6. Anordnung nach Anspruch 5,  d a d u r c h  g e - k e n n z e i c h n e t, dass der Notstopp (7) durch beide Mikroprozessoren 1,2 auslösbar ist.

7. Anordnung nach Anspruch 5 oder 6,  d a d u r c h  g e k e n n z e i c h n e t, dass das Einstellorgan (5) als Dublikat für den Notstopp (7) dient.

8. Anordnung nach einem der Ansprüche 1 bis 7,  d a - d u r c h  g e k e n n z e i c h n e t, dass als Energieversorgung eine ebenfalls dublierte Batterie (10) vorgesehen ist.

9. Anordnung nach Anspruch 8,  d a d u r c h  g e - k e n n z e i c h n e t, dass ein Batterieindikator (11) vorgesehen ist, dessen Signale auf beide Mikroprozessoren (1,2) gegeben sind und beim Ausfall einer Batterie (10) ein Warnsignal verursachen und beim Ausfall beider Batterien zumindest den Notstopp (7) auslösen.

10. Anordnung nach Anspruch 9,  d a d u r c h  g e - k e n n z e i c h n e t, dass der Notstopp (7) eine Hilfsspannungsquelle, insbesondere einen stets aufgeladenen Kondensator, enthält, durch die er auch beim Ausfall beider Batterien (10) betätigbar ist.

11.Anordnung nach einem der Ansprüche 1 bis 10, d a - d u r c h  g e k e n n z e i c h n e t, dass weitere, nicht dublierte Funktionen zumindest teilweise mit Funktionskontrollen versehen sind.

12. Anordnung nach einem der Ansprüche 1 bis 11, d a d u r c h g e k e n n z e i c h n e t, dass mindestens ein Display (13) vorgesehen ist.

13. Anordnung nach einem der Ansprüche 1 bis 12, d a d u r c h g e k e n n z e i c h n e t, dass eine Fernsteuerung (8) vorgesehen ist.

14. Anordnung nach einem der Ansprüche 1 bis 13, d a d u r c h g e k e n n z e i c h n e t, dass ein Speicher (14) vorgesehen ist, in dem mindestens die totale Durchflussmenge zu verschiedenen Zeitpunkten speicherbar ist.

15. Anordnung nach einem der Ansprüche 1 bis 14, d a d u r c h g e k e n n z e i c h n e t, dass ein Blasendetektor (6) vorgesehen ist, gegebenenfalls auch mit Funktionskontrolle oder dubliert, dessen Signal gleichfalls auf beide Mikroprozessoren (1,2) gegeben ist.